# EUROPÄISCHE PATENTSCHRIFT

(11) **EP 1 608 746 B1**
(45) Veröffentlichungstag und Bekanntmachung des Hinweises auf die Patenterteilung: **24.11.2010**
(21) Anmeldenummer: 04711370.9
(22) Anmeldetag: 16.02.2004
(51) Int. Cl.: C12N 9/80, C12N 15/55, C12P 7/42

(54) **THERMISCH STABILE AMIDASEN**
THERMALLY STABLE AMIDASES
AMIDASES THERMIQUEMENT STABLES

(30) Priorität: 21.03.2003 DE 10312842
(43) Veröffentlichungstag der Anmeldung: 28.12.2005
(73) Patentinhaber: Evonik Goldschmidt GmbH, 45127 Essen (DE)
(72) Erfinder: EGOROVA, Ksenia, 21077 Hamburg (DE); ANTRANIKIAN, Garabed, 21218 Hittfeld-Waldesruhd (DE); TRAUTHWEIN, Harald, 80636 München (DE); VERSECK, Stefan, 63456 Hanau (DE); DINGERDISSEN, Uwe, 64342 Seeheim-Jugenheim (DE)
(86) Internationale Anmeldenummer: PCT/EP2004/001430
(87) Internationale Veröffentlichungsnummer: WO 2004/083423

(56) Entgegenhaltungen:
- DATABASE EMBL [Online] EBI; 20. Juli 2001 (2001-07-20) "Bacillus stearothermophilus glutamyl-tRNAGln amidotransferase subunit C (gatC), glutamyl-tRNAGln amidotransferase subunit A (gatA), and glutamyl-tRNAGln amidotransferase subunit B (gatB) genes, complete cds." Database accession no. AY040860 XP002281305
- KOBAYASHI M ET AL: "AMIDASE COUPLED WITH LOW-MOLECULAR-MASS NITRILE HYDRATASE FROM RHODOCOCCUS RHODOCHROUS J1. SEQUENCING AND EXPRESSION OF THE GENE AND PURIFICATION AND CHARACTERIZATION OF THE GENE PRODUCT" EUROPEAN JOURNAL OF BIOCHEMISTRY, BERLIN, DE, Bd. 217, 1993, Seiten 327-336, XP000652066 ISSN: 0014-2956
- D'ABUSCO ANNA SCOTTO ET AL: "Molecular and biochemical characterization of the recombinant amidase from hyperthermophilic archaeon Sulfolobus solfataricus" EXTREMOPHILES, Bd. 5, Nr. 3, Juni 2001 (2001-06), Seiten 183-192, XP002281301 ISSN: 1431-0651
- NAWAZ M S ET AL: "Physical, biochemical, and immunological characterization of a thermostable amidase from Klebsiella pneumoniae NCTR 1." JOURNAL OF BACTERIOLOGY. UNITED STATES APR 1996, Bd. 178, Nr. 8, April 1996 (1996-04), Seiten 2397-2401, XP002281302 ISSN: 0021-9193
- BAEK DAE HEOUN ET AL: "New thermostable D-methionine amidase from Brevibacillus borstelensis BCS-1 and its application for D-phenylalanine production." ENZYME AND MICROBIAL TECHNOLOGY, Bd. 32, Nr. 1, 2. Januar 2003 (2003-01-02), Seiten 131-139, XP002281303 ISSN: 0141-0229
- YAMAKI T ET AL: "CLONING AND SEQUENCING OF A NITRILE HYDRATASE GENE FROM PSEUDONOCARDIA THERMOPHILA JCM3095" JOURNAL OF FERMENTATION AND BIOENGINEERING, SOCIETY OF FERMENTATION TECHNOLOGY, JP, Bd. 83, Nr. 5, 1997, Seiten 474-477, XP002050253 ISSN: 0922-338X
- DATABASE GENBANK PROTEIN [Online] NIH; 6. Juni 2002 (2002-06-06) PARKHILL, J. ET AL.: "Putative DNA helicase [Salmonella enterica subsp. enterica serovar Typhi]" Database accession no. CAD06784 XP002281306 & PARKHILL, J. ET AL.: "Complete genome sequence of a multiple drug resistant salmonella enterica serovar typhi CT18" NATURE, Bd. 413, 25. Oktober 2001 (2001-10-25), Seiten 848-852, XP002965014
- EGOROVA ET AL.: "Purification and properties of an enantioselective and thermoactive amidase from the thermophilic actinomycete Pseudonocardia thermophila" APPLIED MIRCORBIOLOGY AND BIOTECHNOLOGY [EPUB AHEAD OF PRINT], [Online] 21. April 2004 (2004-04-21), Seiten 1-15, XP002281304 Gefunden im Internet: <URL:http://springerlink.metapress.com/med ia/D86TGDPAFP7WWG98TY47/Contributions/J/A/ F/Y/JAFY3JRJ0C6XF985_html/fulltext.html> [gefunden am 2004-05-24]
- WHITE O ET AL: "GENOME SEQUENCE OF THE RADIORESISTANT BACTERIUM DEINOCOCCUS RADIODURANS R1" SCIENCE, AMERICAN ASSOCIATION FOR THE ADVANCEMENT OF SCIENCE, WASHINGTON, DC; US, vol. 286, 1 January 1999 (1999-01-01), pages 1571-1577, XP002254144 ISSN: 0036-8075 -& DATABASE UniProt [Online] 1 May 2000 (2000-05-01), "SubName: Full=Beta-phosphoglucomutase-related protein;" retrieved from EBI accession no. UNIPROT:Q9RTX8 Database accession no. Q9RTX8
- WHITE O ET AL: "GENOME SEQUENCE OF THE RADIORESISTANT BACTERIUM DEINOCOCCUS RADIODURANS R1" SCIENCE, AMERICAN ASSOCIATION FOR THE ADVANCEMENT OF SCIENCE, WASHINGTON, DC; US, vol. 286, 1 January 1999 (1999-01-01), pages 1571-1577, XP002254144 ISSN: 0036-8075 -& DATABASE UniProt [Online] 1 May 2000 (2000-05-01), "SubName: Full=Beta-phosphoglucomutase-related protein;" retrieved from EBI accession no. UNIPROT:Q9RTX8 Database accession no. Q9RTX8

### SEQUENZPROTOKOLL

<110> Degussa AG
<120> Thermisch stabile Amidasen
<130> 202dg07.de
<140>
   <141>
<150> DE 103 12 842
   <151> 2003-03-21
<160> 4
<170> PatentIn Ver. 2.1
<210> 1
   <211> 9
   <212> PRT
   <213> Pseudonocardia thermophila
<400> 1
   Ile His Met Pro Asp Pro Asp Ala Val
     1               5
<210> 2
   <211> 14
   <212> PRT
   <213> Pseudonocardia thermophila
<400> 2
   Asp Gly Leu Pro Val Gly Leu Met Ile Val Gly Lys His Phe
     1               5                  10
<210> 3
   <211> 509
   <212> PRT
   <213> Pseudonocardia thermophila
<400> 3
   Met Ile His Met Pro Asp Pro Asp Ala Val Ala Arg Met Ala Ala Glu
   1 5 10 15
   Gly Arg Phe Gly Ile Pro Val Val Val Ile Cys Arg Thr Tyr His Asp
           20                  25                  30
   Leu Gly His Arg Pro Ala Arg Leu Val Glu Gln Gly Arg Arg Ala Val
               35                  40                  45
   Arg Gly Gly Gly Pro His Pro Ala Ala Ala Leu Val Asp Pro Pro Arg
       50                  55                  60
   Arg Arg Gly Arg Gln Ala Arg Arg Val Gly Gly Ala Asp Leu Asp His
   65                  70                  75                  80
   Arg Asn Leu Gly Gly Ala Ala Arg Arg Pro His Gly Arg Gly Gln Asp
                   85                  90                  95
   Asn Val Ala Val Ala Gly Val Pro Met Met Asn Gly Ser Arg Thr Leu
               100                 105                 110
   Glu Gly Phe Val Pro Ala Glu Asp Ala Thr Val Val Ser Arg Leu Leu
           115                 120                 125
   Ala Ala Gly Ala Thr Ile Ala Gly Lys Ser Val Cys Glu Asp Leu Cys
       130                 135                 140
   Phe Ser Gly Gly Ser His Thr Ser Lys Pro Gly Thr Val His Asn Pro
   145                 150                 155                 160
   Trp Asp Met Ser Arg Ser Ala Gly Gly Ser Ser Ser Gly Ser Gly Ala
                   165                 170                 175
   Leu Val Ala Ala Gly Glu Val Asp Met Ala Ile Gly Gly Asp Gln Gly
               180                 185                 190
   Gly Ser Ile Arg Ile Pro Ser Ala Tyr Cys Gly Thr Val Gly His Lys
           195                 200                 205
   Pro Thr His Gly Leu Val Pro Tyr Thr Gly Gly Phe Pro Ile Glu Gln
       210                 215                 220
   Ser Ile Asp His Leu Gly Pro Ile Thr Arg Thr Val Ala Asp Ala Ala
   225                 230                 235                 240
   Leu Met Leu Ser Val Ile Ala Gly Arg Asp Gly Leu Asp Pro Arg Gln
                   245                 250                 255
   Pro Asp Val Val Glu Val Gln Asp Tyr Val Gly Ala Leu Ala Glu Ser
               260                 265                 270
   Val Ser Gly Leu Arg Ile Gly Val Leu Gln Glu Gly Phe Gly His Pro
   275 280 285
   Asn Ser Glu Pro Glu Val Asp Asp Thr Val Arg Ala Ala Val Gly Thr
   290 295 300
   Leu Arg Glu Ala Gly Phe Thr Val Glu Asp Val Ser Val Pro Trp His
   305 310 315 320
   Leu His Ala Thr Ala Ile Trp Asp Val Leu Ala Thr Glu Gly Gly Leu
   325 330 335
   Trp Gln Met Val Glu Gly Asn Ala Tyr Gly Met His Trp Lys Gly His
   340 345 350
   Tyr Asp Pro Ser Leu Ile Ala Tyr Tyr Gly Arg Lys Trp Arg Glu Asp
   355 360 365
   Pro Ala Gln Phe Ser Glu Thr Val Lys Leu Val Ala Leu Ala Gly Arg
   370 375 380
   Tyr Ala Leu Thr Thr Gln Tyr Gly Arg His Tyr Ala Met Ala Arg Asn
   385 390 395 400
   Leu Ala Pro Lys Leu Val Ala Ala Tyr Asp Ala Ala Leu Ser Asn Tyr
   405 410 415
   Asp Val Leu Val Met Pro Thr Leu Pro Met Arg Ala Thr Val Leu Pro
   420 425 430
   Gly Pro Asp Ala Pro Val Glu Glu Ile Leu Ala Arg Gly Leu Glu Met
   435 440 445
   Leu Ala Asn Thr Ala Pro Phe Asp Val Thr Gly His Pro Ala Cys Ser
   450 455 460
   Val Pro Ala Gly Leu Ala Asp Gly Leu Pro Val Gly Leu Met Ile Val
   465 470 475 480
   Gly Lys His Phe Asp Asp Ala Thr Val Leu Lys Val Ala Asn Ala Phe
   485 490 495
   Glu Gln Ala Val Gly Gly Phe Pro Thr Pro Ala Ala Glx
   500 505
<210> 4
   <211> 1527
   <212> DNA
   <213> Pseudonocardia thermophila
<400> 4
   atgatccaca tgccggaccc ggacgcggtg gcccggatgg ccgcagaggg tcggttcggg 60
   atccccgtag tagtgatctg ccgtacctac cacgacctcg gtcaccggcc tgctcggctc 120
   gtggaacaag gtcgaagagc tgtacgcgga ggtggccccc accccgccgc agcgctcgtg 180
   gacccgcccc gacgccgagg acgacaagct cggcgcgtgg gcggtgcaga cctcgatcac 240
   cgaaacctcg gaggggccgc tcgccggccg cacggtcgcg gtcaggacaa cgtcgccgtc 300
   gccggcgtgc cgatgatgaa cggatcgcgg accctggagg gcttcgtccc cgccgaggac 360
   gccaccgtcg tgtcccggct gctcgccgcc ggcgcgacca tcgccggcaa gtcggtgtgc 420
   gaggacctgt gcttctccgg cggctcgcac accagcaagc cgggaaccgt gcacaacccg 480
   tgggacatga gccgctcggc gggtggttcc tcgagcggta gcggggcgct ggtggccgcc 540
   ggcgaggtcg acatggccat cggcggtgac cagggcgggt cgatccgcat cccgtcggcg 600
   tactgcggca ccgtcgggca caagccgacc cacggcctgg tgccgtacac cggaggcttc 660
   ccgatcgagc agtcgatcga ccacctcggc ccgatcaccc gcaccgtcgc cgacgccgcg 720
   ctcatgctgt cggtgatcgc cggccgcgac gggctcgacc cgcgccagcc cgacgtcgtc 780
   gaggtgcagg actacgtcgg cgcgctggcc gagtcggtga gcgggctgcg catcggcgtc 840
   ctgcaggagg gcttcggtca ccccaactcc gagcccgagg tcgacgacac cgtgcgcgct 900
   gctgtcggga cactgcgcga ggccggcttc accgtcgagg acgtctcggt cccgtggcac 960
   ctgcacgcca cggccatctg ggacgtgctc gccaccgagg gcggcctgtg gcagatggtc 1020
   gagggcaacg cctacggcat gcactggaag ggccactacg acccgtcgct catcgcctac 1080
   tacgggcgca agtggcggga ggacccggcg cagttctccg agacggtcaa gctcgtcgcc 1140
   ctcgccggcc gctacgcgct cacgacccag tacgggcgcc actacgcgat ggcgcgcaac 1200
   ctcgccccga agctggtcgc ggcctacgac gcggcgctgt cgaactacga cgtgctcgtc 1260
   atgccgacgc tgccgatgcg cgccaccgtg ctgcccggcc cggacgcgcc cgtcgaggag 1320
   atcctcgcca ggggcctgga gatgctggcc aacaccgcac cgttcgacgt caccgggcac 1380
   ccggcctgct ctgtcccggc cggcctcgcc gacggtctcc cggtcggcct gatgatcgtc 1440
   ggcaagcact tcgacgacgc caccgtgctg aaggtggcga acgccttcga acaggccgtc 1500
   ggcggcttcc cgacccccgc tgcctga                                      1527

## Beschreibung

Die Erfindung betrifft neue Amidasen, die aus thermophilen Bakterien isoliert werden können, insbesondere aus thermophilen Actinomyceten, wie z. B. *Pseudonocardia thermophila,* wobei die erhaltenen Amidasen nicht nur eine hohe Temperaturstabilität besitzen, sondern sich auch durch die enantioselektive Umsetzung eines breiten Substratspektrums auszeichnen.

Enzymatische Verfahren halten zunehmenden Einzug in der Organischen Synthese, insbesondere bei der Durchführung enantioselektiver Synthesen, wie z. B. bei der Herstellung von optisch reinen pharmazeutischen Wirkstoffen, Aminosäuren, Acrylsäuren oder Hydroxamsäuren. Weiterhin sind Amidasen bei dem Abbau von xenobiotischen Verbindungen beteiligt.

Amidasen katalysieren dabei die Umsetzung von Amiden zu ihren korrespondierenden Carbonsäuren und Aminen bzw. zu Ammoniak. Inzwischen konnten eine Reihe von bakteriellen Amidasen identifiziert werden, darunter auch einige Amidasen aus mesophilen Actinomyceten (Bhalla, T.C., et al.; Science letters 11-12; 139-141, 1997; Hirrlinger, B.; et al. J. Bacteriol. 178, 3501-3507, 1996; Kobayashi, M., et al.; Eur. J. Biochem., 217, 327-336, 1993 ; Kotlova, E. K.; et al. ; Biochemistry (Mosc.) 64, 384-389, 1999 ; Mayaux, J.F. et al. ; J. Bacteriol. 173, 6694-6704, 1991; Mayaux, J.F.; J. Bacteriol. 172, 6764-6773, 1990; Nawaz, M.S., et al.; Appl. Environ. Microbiol., 60, 3343-3348, 1994), wobei allerdings keine thermostabile Amidase aus thermophilen Actinomyceten beschrieben wurde.

Thermoaktive Amidasen wurden demgegenüber bisher nur in *Klebsiella pneumoniae* NCTR 1 (Nawaz, M.S.; J. Bacteriol. 178, 2397-2401, 1996) und in *Sulfolobus solfataricus* (d'Abusco, A.S.; Extremophiles, 5, 183-192, 2001) gefunden. Gerade die Bereitstellung von weiteren Amidasen, insbesondere von thermostabilen Amidasen wäre allerdings von großem technischen Interesse, da sich mit solchen Enzymen ein breiteres Spektrum an technisch durchführbaren enzymatischen Umsetzungen erschließt. Der vorliegenden Erfindung lag daher Aufgabe zu Grunde neue, thermisch stabile Amidasen bereit zu stellen.

Die Aufgabe wird durch Amidasen gemäß Anspruch 1 gelöst, die eine Aminosäuresequenz gemäß **SEQ ID No. 3** besitzt oder die eine Aminosäuresequenz mit einer Homologie von mindestens 80 % zu SEQ ID No. 3 besitzt und die eine N-terminale Sequenz **(SEQ ID No. 1)**
I H M P D P D A V **(SEQ ID No. 1)**
und / oder eine Aminosäuresequenz
D G L P V G L M I V G K H F **(SEQ ID No. 2)**
enthalten.

Die neuen Amidasen sind z. B. aus thermophilen Bakterien, insbesondere aus thermophilen Actinomyceten, wie z. B. aus *Pseudonocardia thermophila* erhältlich. So kann eine in *Pseudonocardia thermophila* konstitutiv exprimierte Amidase mit einem Molekulargewicht zwischen 47 und 53 kDa isoliert werden, die bevorzugt als Dimer mit einem Molekulargewicht zwischen 100 und 140 kDa auftritt.
Die Identifikation weiterer mutanter oder alleler Varianten der Amidasen kann z. B. anhand von Nukleinsäuresonden erfolgen, die zu einer DNA-Sequenz, codierend für die Aminosäuresequenz **SEQ ID No. 1** oder **SEQ ID No. 2,** komplementär sind. Die Hybridisierung einer solchen Sonde erfolgt dabei unter stringenten Bedingungen, z. B. bei 60°C, 0,1xSSC, 0.1% SDS.

Die gefundenen nativen Amidasen können zwar durch die N-terminale Sequenz **SEQ ID No. 1** und die **SEQ ID No. 2** oder einer homologen Variante davon identifiziert werden, aber für die Amidaseaktivität ist zumindest die N-terminale Sequenz nicht zwingend erforderlich. Daher umfasst die vorliegende Erfindung auch solche, oben beschriebenen Amidasen, deren N-terminales Ende künstlich deletiert ist. Ebenso kann auch die Teilsequenz **SEQ ID No. 2** künstlich deletiert werden.

Ein weiterer Gegenstand der vorliegenden Erfindung stellen Nukleinsäuren codierend eine erfindungsgemäße Amidase mit einer Sequenz gemäß **SEQ ID No. 4,** oder mit einer Nukleotidsequenz mit einer Homologie von über 90 % dazu, dar. **SEQ ID No. 4** codiert eine Amidase mit einer Aminosäuresequenz **SEQ ID No. 3.**

Die beschriebenen Amidasen können aus einem zellfreien Rohextrakt thermophiler Bakterien, der z. B. durch Ultraschallaufschluss von *Pseudonocardia thermophila* Zellen in einem Phosphatpuffer erhältlich ist, wie folgt gereinigt werden:
a) Zentrifugation des Rohextraktes bei 10000 bis 20000 rpm und anschließende Zugabe einer 1 M Salzlösung, bevorzugt einer KCl-Lösung,
b) chromatographische Trennung des Überstandes an einer hydrophoben Säule mit einem reversen Gradienten einer Salzlösung, bevorzugt einer KCl-Lösung, von 1 M bis 0 M,
c) Ultrafiltration der aus b) erhaltenen Fraktion, die Amidaseaktivität zeigt, an einer 10 kDa cut off Membran,
d) lonenaustauschchromatographie der aus c) erhaltenen Proteinfraktion mit einem Gradienten von 0 M bis 0,5 M einer Salzlösung, bevorzugt einer NaCl-Lösung
e) Chromatographie der aus d) erhaltenen Fraktion, die Amidaseaktivität zeigt, mit einer 0,1%igen Salzlösung, bevorzugt einer 150 mM NaCl-Lösung, und entsalzen der gereinigten Amidasefraktion.

Bevorzugt werden Alkalihalogenid-Salzlösungen, wie z. B. NaCl oder KCI - Lösungen zur Durchführung der einzelnen chromatographischen Reinigungsschritten verwendet. Die chromatographischen Trennungen werden bevorzugt zwischen pH 6,5 und 8,0 durchgeführt, wobei der pH-Wert, z. B. durch Verwendung eines Standard Phosphatpuffers (pH 7,2) eingestellt werden kann. Die lonenaustauschchromatographie wird bevorzugt bei einem pH-Wert zwischen 7,5 und 8,5 durchgeführt.

Die Amidaseaktivität der erhaltenen Fraktionen kann mit Hilfe eines Hydrolyse-Tests bestimmt werden, wobei als Substrat Benzamid verwendet werden kann. Die enzymatisch entstehenden Produkte, Benzoesäure und Ammoniak, können mittels HPLC (Benzoesäure) bzw. mit der Phenyl-hydrochlorid Methode (Nachweis von Ammoniumionen) bestimmt werden.

Die so gewonnenen erfindungsgemäßen Amidasen zeichnen sich durch eine hohe Temperaturstabilität aus. Die spezifische Aktivität des Enzyms leidet bei kürzeren Reaktionszeiten von mehr als einer Stunde erst bei Temperaturen um 80 °C signifikant. Weiterhin besitzen die beschriebenen Amidasen ein Temperaturoptimum zwischen 50 und 75 °C. Die beschriebenen Amidasen zeigen darüber hinaus eine nennenswerte Aktivität zwischen 30 und 85 °C, bevorzugt sind die Enzyme allerdings bei Reaktionstemperaturen zwischen 60 und 70 °C verwendbar.

Weiterhin bleibt auch die Spezifische Aktivität der erfindungsgemäßen Amidasen über einen weiten pH-Wertbereich erhalten. So besitzen die Amidasen eine signifikante enzymatische Aktivität zwischen pH 3,5 und pH 11,5, wobei die spezifische Aktivität vom Optimum zwischen pH 6,0 und 7,5 über den Bereich von pH 4,5 und 10,0 kaum abfällt.

Durch das hohe Temperaturoptimum, die gute thermische Stabilität und den großen pH-Wertbereich in dem die Enzyme aktiv sind, erschließt sich ein interessanter Spielraum für die Optimierung Amidase katalysierter Prozesse, wie der Hydrolyse von Amiden.

Es kann darüber hinaus gezeigt werden, dass die hier beschriebenen Amidasen unempfindlich gegenüber einer großen Zahl von Reagenzien und Ionen sind. So sind die Amidasen z. B. unempfindlich gegenüber Chelatbildnern wie z. B. EDTA oder gegenüber Detergenzien wie SDS oder Triton. Auch die Abhängigkeit von ionischen Co-Faktoren scheint nicht gegeben zu sein. Durch Zugabe von DTT kann sogar die enzymatische Aktivität der Amidasen gesteigert werden.

Die gewonnenen und charakterisierten erfindungsgemäßen Amidasen unterscheiden sich in ihren Eigenschaften deutlich von den bisher bekannten mikrobiellen Amidasen, wie aus der exemplarischen Zusammenstellung in Tab. 4 ersichtlich ist. Es ist dabei insbesondere bemerkenswert, dass das isolierte Enzym aus *Pseudonocardia thermophila* die erste bekannte natürlich vorkommende homotrimere Amidase ist.

**Tab. 4**

| Mikroorganismus | Charakterisitka | | | | | | | |
|---|---|---|---|---|---|---|---|---|
| | Expression | Molmasse in kDa | Untereinheiten kDa (Anzahl) | pH-Opt. | T-Opt. in °C | IEF Punkt pH | Metall Co-Faktoren | Substratspezifität (Amide) |
| *Pseudonocardia thermophila* | konstitutiv | 110 | 50 (2) | 7.0 | 70 | 4.2 | n.d. | Al, Ar, Cy, As |
| | | | | | | | | |
| *Agrobacterium tumefaciens* d3 ^{a)} | induzierbar | 490 | 63 (8) | 7.5 | 22 | n.d. | n.d. | Al, Ar |
| *Bacillus stearothermophilus* ^{b)} | induzierbar | 39 | monomer | 7.0 | 55 | n.d. | n.d. | Al |
| *Brevibacterium sp.* R312 ^{c)} | induzierbar | 110 | 43 (2) | 7.5 | 30 | n.d. | n.d. | Arylpropioamid |
| *Comamonas acidovorans* KPO ^{d)} | konstitutiv | 54 | monomer | 8.0 | 30 | n.d. | n.d. | Ketoprofen |
| DSM 6320 ^{e)} | induzierbar | 125 | 66 (2) | 8.5 | 40 | 4.2 | n.d. | L-Carnitin |
| *Helicobacter pylori* ^{f)} | konstitutiv | 40 | monomer | 7.0 | 55 | n.d. | n.d. | Al |
| *Klebsiella pneumoniae* NCTR1 ⁹⁾ | induzierbar | 62 | monomer | 7.0 | 65 | n.d. | Co&Fe | Al |
| *Mycobacterium neoaurum* ^{h)} | konstitutiv | 136 | 40 (4) | 8.0 | 50 | 4.2 | n.d. | Al, As |
| *Mycobacterium smegmatis* ⁱ⁾ | konstitutiv | 50 | monomer | 7.5 | 22 | n.d. | n.d. | Cy |
| *Ochrobactrum antropi* SV3 ^{j)} | induzierbar | 40 | 63 (8) | 9.0 | 22 | n.d. | n.d. | As |
| *Pseudomonas aeruginosa* ^{k)} | induzierbar | 200 | monomer | 7.0 | 55 | n.d. | n.d. | Al |
| *Pseudomonas chlororaphis* B23 ^{l)} | induzierbar | 105 | 54 (2) | 7.0 | 50 | n.d. | no metal | Al, Ar, Cy, As |
| *Rhodococcus rhodochrous* J1 ^{m)} | induzierbar | 110 | 55 (2) | 7.9 | 55 | n.d. | n.d. | Al, Ar, Cy, As |
| *Rhodococcus rhodochrous* M8 ⁿ⁾ | konstitutiv | 150 | 42 (4) | 7.0 | 55 | n.d. | n.d. | Al |
| *Rhodococcus erythropolis* MP50 ^{o)} | induzierbar | 480 | 61 (8) | 7.5 | 55 | n.d. | n.d. | Al, Ar, Cy |
| *Rhodococcus sp.* ^{p)} | konstitutiv | 360 | 44,5 (8) | 8.5 | 40 | 4.0 | Fe | Al |
| *Rhodococcus sp.* NHB-2 ^{q)} | induzierbar | n.d. | n.d. | 8.0 | 55 | n.d. | n.d. | Al |
| *Rhodococcus sp.* ^{r)} | induzierbar | 118 | 48,5 (2) | 7.5 | 30 | n.d. | n.d. | Arylpropionamid |
| *Stenotrophomonas maltophila* ^{s)} | induzierbar | 38 | monomer | 6.0 | 40 | 5.8 | n.d. | As, Peptide |
| *Sulfolobus solfataricus* ^{t)} | induzierbar | 56 | monomer | 7.5 | 95 | 5.94 | n.d. | Al, Ar, Cy |

| | | | | | | | | |
|---|---|---|---|---|---|---|---|---|
| Al = Aliphatische Amide, Ar = Aromatische Amide, Cy = Cyclische Amide, As = Aminosäureamide, n.d. = nicht bestimmt a) Trott et al.; Mikrobiology 147, 1815-1824, 2001 ; b) Cheong et al., Enzyme Microb. Technol. 26, 152-158, 2000 ; c) Mayaux et al., J. Bacteriol. 172, 6764-6773, 1990 ; d) Yamamoto et al., Appl. & Environ. Microbiol. 1, 152-155, 1996; e) Joeres et al., Appl. Microbiol. Biotechnol. 40, 606-610, 1994, f) Skouloubris, et al., Mol. Microbiol. 25, 989-998, 1997 ; g) Nawaz et al., J. Bacteriol. 178, 2397-2401, 1996 ; h) Hermes et al., Appl. & Environ. Microbiol. 1, 153-159, 1994; i) Boshoff et al., J. Bacteriol. 180, 5809-5814, 1998; j) Komeda et al., Eur. J. Biochem. 267, 2028-2035, 2000 ; k) Nawaz et al., Appl. Biochem. & Biotechnol. 28/29, 865-875, 1991; l) Ciskanik et al., Appl. Environ. Microbiol. 61, 998-1003, 1995, m) Kobayashi et al., Eur. J. Biochem., 217, 327-336, 1993 ; n) Kotlova et al., Biochemistry (Mosc), 64, 384-389, 1999 ; o) Hirrlinger et al., J. Bacteriol. 178, 3501-3507, 1996 ; p) Nawaz et al., Appl. Envirion. Microbiol. 60, 3343-3348, 1994; q) Bhalla et al., Science letters, 11-12, 139-142, 1997 ; r) Mayaux et al., J. Bacteriol., 173, 6694-6704, 1991 ; s) Neumann et al., Appl. Microbiol. & Biotechnol. 58, 773-780, 2002; t) d'Abusco et al., Extremophiles, 5, 183-192, 2001 | | | | | | | | |

Ein großer Vorteil der neuen Amidasen ist deren breites Substratspektrum. So lassen sich mit den Enzymen sowohl aliphatische, aromatische, cyclische, heterocyclische und Aminosäureamide hydrolysieren. Wobei insbesondere aliphatische Amide mit ein bis zehn Kohlenstoffatomen, aromatische Amide mit 5 bis 12 Kohlenstoffatomen, heterocyclische Amine mit 4 bis 10 Kohlenstoffatomen und mit ein bis vier Heteroatomen, z. B. ausgewählt aus der Gruppe N, S, O, P oder L-Aminosäureamide umgesetzt werden können.

Die beschriebenen Amidasen zeichnen sich neben dem breiten Substratspektrum auch durch eine hohe Enantioselektivität aus. Insbesondere die Herstellung von S-Enantiomeren, wie z. B. von (hetero-)cyclischen Säuren und insbesondere aromatischen, aliphatischen oder aromatisch-aliphatischen Säuren, ist bevorzugt.

### Kurze Beschreibung der Figuren:

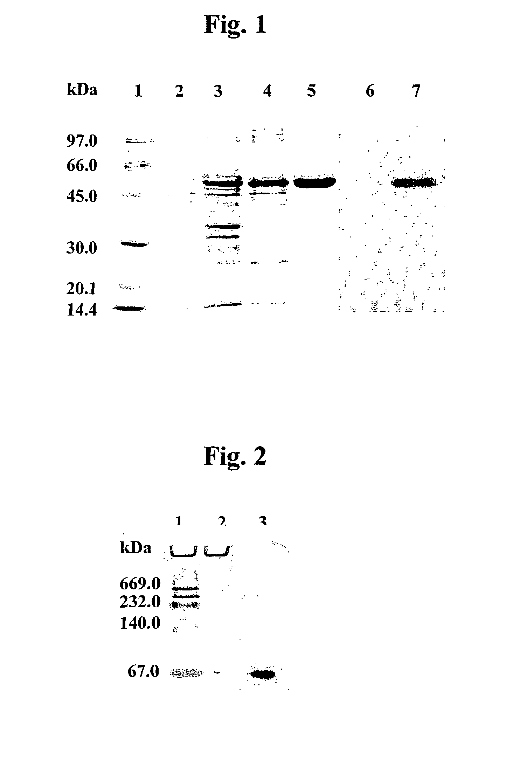

Fig. 1 zeigt die Ergebnisse einer SDS-PAGE Elektrophorese mit unterschiedlichen Enzymproben, die während der Aufreinigung entnommen wurden (Bahnen 2 bis 5). Die Bahnen 6 und 7 zeigen ein Zymogram des Rohextraktes und der aufgereinigten Probe. Bahn 1 ist ein Molekulargewichtsstandard.

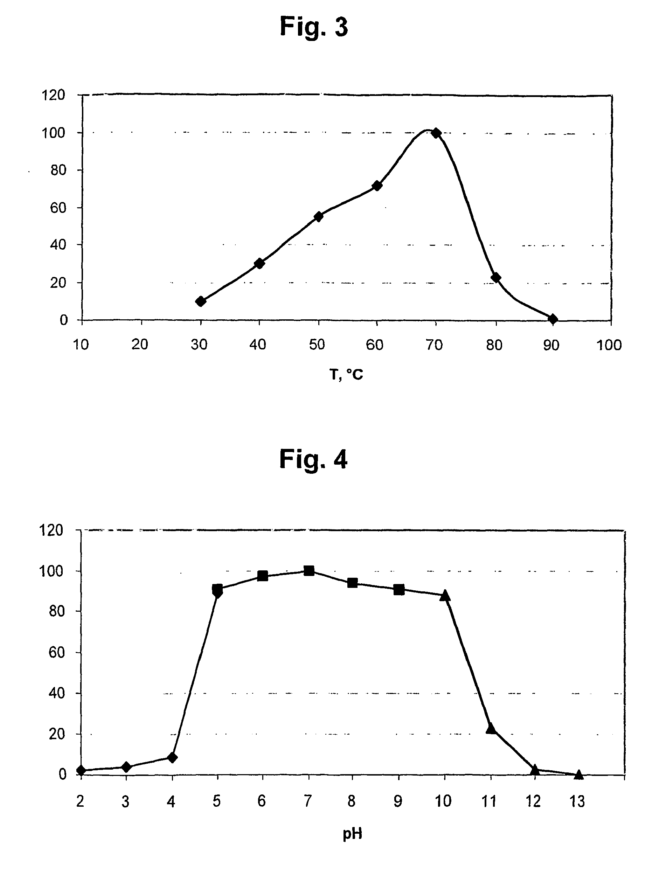

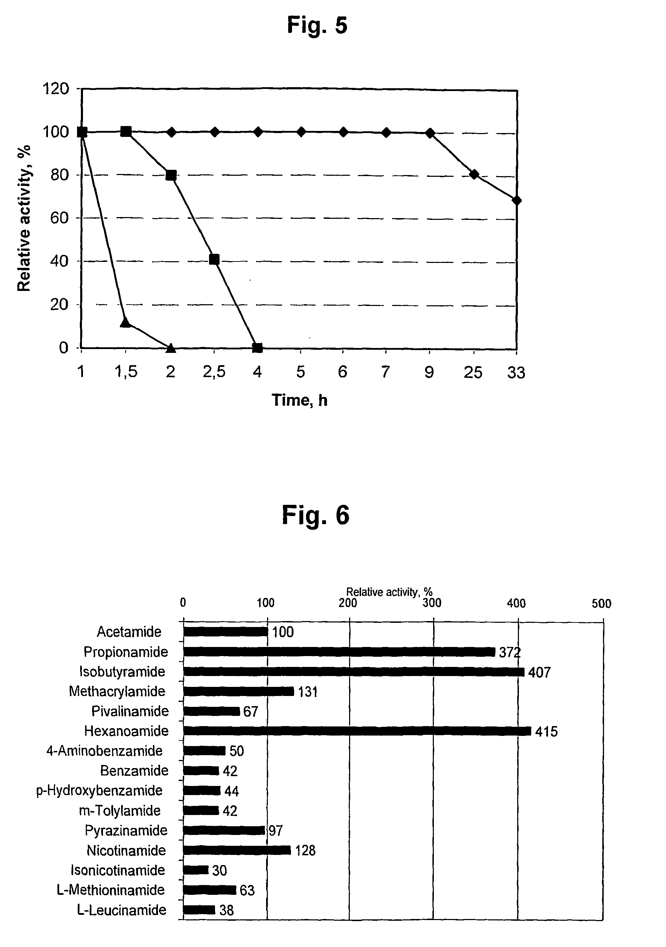

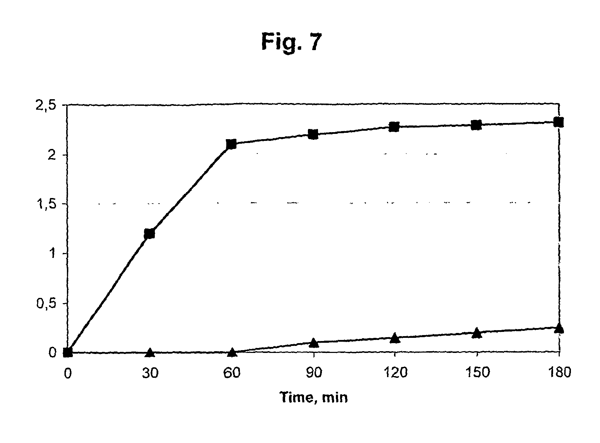

Fig. 2 zeigt das Ergebnis der Molekulargewichtsbestimmung der nativen Amidase mittels Gelfiltration. Fig. 3 zeigt die Spezifische Aktivität einer erfindungsgemäßen, gereinigten Amidase in Abhängigkeit von der Reaktionstemperatur, Fig.4 in Abhängigkeit vom pH-Wert. Fig. 5 zeigt die Stabilität der gereinigten Amidase bei unterschiedlichen Temperaturen. In Fig. 6 ist die Acetyltransferase-Aktivität bezogen auf beispielhafte Substrate wiedergegeben. In Fig. 7 ist die enantioselektive enzymatische Umsetzung mit 2-Phenylpropionamid als Substrat gezeigt.

Im folgenden werden einige Ausführungsbeispiele gegeben, die allerdings nicht einschränkend zu verstehen sind.

### Beispiel 1: Kultivierung von Pseudonocardia thermophila (DSMZ 43832)

Der verwendete *Pseudonocardia thermophila* Stamm wurde von der Deutschen Sammlung von Mikroorganismen und Zellkulturen (DSMZ, Braunschweig, Germany) bezogen. Die Zellen wurden, sofern nichts anderweitig erwähnt ist, wie in Yamaki, T.; et al.; J. Ferment. Bioeng., 83, 474-477, 1997 beschrieben, kultiviert. Das Medium (pH 7.2) enthält pro Liter: 5g Hefeextrakt, 4g lösliche Stärke, 0,3g KH₂PO₄, 0,6g Na₂HPO_{4,} 0,1g M₉SO₄ • 7H₂O, 5g NaCl. Die Zellen wurden nach 3 Tagen Kultivierung in einem Schüttelkolben bei 50 °C, 150 rpm geerntet. Die Ausbeute betrug 10g Zellen (Nassgewicht) pro Liter Medium.

### Beispiel 2: Reinigung der Amidase:

Die Aufreinigung der Amidase erfolgte bei Raumtemperatur. 7g Zellmaterial (Nassgewicht) wurden dazu 2 mal mit 60 mM K-Na Phosphatpuffer (Standardpuffer), pH 7.2 gewaschen, in 50 ml des Puffers resuspendiert und mittels Ultraschall aufgeschlossen. Nach 20-minütiger Zentrifugation bei 13000rpm, wurde eine 1 M KCl-Lösung zu dem zellfreien Extrakt gegeben. Der Überstand (50ml) wurde auf eine hydrophobe Phenyl-Sepharose Fast Flow Säule (Pharmacia, Sweden) gegeben, die mit einem 1 M KCl Standardpuffer equilibriert war. Das Protein wurde dann mit einem linearen reversen Gradienten einer 1 M KCI bis 0 M KCl -Lösung eluiert. Die Amidase enthaltenden Fraktionen wurden vereinigt und gegen 2 L Standardpuffer (pH 8,0) dialysiert. Anschließend wurde die erhaltene Proteinfraktion auf das 12-fache konzentriert und durch eine 10 kDa cut-off Membran (Amicon) ultrafiltriert. Die Probe wurde dann über eine lonenaustausch-Chromatographie weiter gereinigt. Dazu wurde eine UNO-Q12 Säule (BioRad) verwendet, wobei das gereinigte Protein mit Hilfe eines linearen NaCl-Gradienten (0 bis 0,5 M NaCl in Standardpuffer pH 8,0) gereinigt wurde. Die enzymatisch aktiven Fraktionen wurden vereinigt, 2-fach konzentriert und über eine preparative HiLoad 26/60 Superdex 200 Säule (Pharmacia, Sweden), mit Standardpuffer, pH 7,2, enthaltend 150 mM NaCl, fraktioniert. Die Proteinfraktion mit Amidaseaktivität wurden gesammelt und über eine Sephadex PD-10 Säule (Pharmacia, Sweden) entsalzt. Die Proteinkonzentration wurde mit Rinderserumalbumin als Standard, wie in Bradford, M.M.; Anal. Biochem. 71, 248-254, 1976 beschrieben, bestimmt.

Das Ergebnis der Reinigung ist in Figur 1 und Tabelle 1 dargestellt.

Der Reinigungserfolg wurde dabei mit einer sodium dodecyl slphatepolyacrylamide (SDS-PAGE) Gelelktrophorese überprüft. Dazu wurde ein vorgefertigtes Gel (Novex, Invitrogen, Niederlande) mit einem Tris-Glycin Gradienten von 4-20% verwendet. Als Standardproteine wurden Phosphorilase b (94kDa), Bovine Albumin (67kDa), Chicken Ovalbumin (43kDa), Carbonic Anhydrase (30kDa), Soybean Trypsin Inhibitor (20.1kDa) und Bovine α-Lactalbumin (14.4kDa) (Pharmacia, Schweden) verwendet. Die Proteinbanden wurden mit Coomassie Blue R-250 angefärbt.

Um die Amidaseaktivität auf dem Gel zu verifizieren wurde das SDS-Gel in 2,5% Triton X-100 60 Minuten gewaschen und anschließend 30 Minuten bei 60 °C in K-Na-Phosphat Puffer (pH 7,0) enthaltend 70 mM Propionamid und 0,7 M Hydroxylaminhydrochlorid, wobei der pH-Wert mit 10 M NaOH auf 7,0 eingestellt wurde, inkubiert. Das Gel wurde danach in Wasser für 2-3 sec. gewaschen und mit einer sauren Eisenchlorid-Lösung (0.1 M FeCl₃ in 0.5 M HCl) bedeckt.

Das aufgetragene Eisen reagiert mit der enzymatisch entstehenden Hydroxamsäure unter Bildung einer rot-braunen Bande. Die Intensität der Färbung entspricht der enzymatischen Aktivität der Amidase. Der eben beschriebene Aktivitätstest wird im folgenden in der Beschreibung auch als Zymogram bezeichnet.

Fig. 1 zeigt die Ergebnisse einer SDS-PAGE Elektrophorese mit unterschiedlichen Proben (jeweils 5 µg Protein), aus den während der Reinigung erhaltenen Fraktionen. Die Proteine wurden mit Coomassie Blue angefärbt. Die Bahn 1 zeigt die Standardproteine, Bahn 2 den Rohextrakt, Bahn 3 eine Probe nach Reinigung an der Phenyl-Sepharose Säule, Bahn 4 eine Probe nach Ionenaustausch-Chromatographie an einer UNO Q12 Säule, Bahn 5 eine Probe nach Reinigung an einer Superdex 200 Säule. Die Bahnen 6 und 7 zeigen das Zymogram des Rohextraktes und der aufgereinigten Probe.

**Tab.1: Ergebnisse zur Reinigung der Amidase aus Pseudonocardia thermophila.**

| **Reinigungsschritt** | **Protein in mg (gesamt)** | **Gesamtaktivität (U)^{a}** | **Spezifische Aktivität (U)** | **Ausbeute (U x mg⁻¹)** | **Reinigungsgrad** |
|---|---|---|---|---|---|
| Rohextrakt ^{b} | 85 | 34.7 | 0.4 | 100 | 1-fach |
| Phenyl-Sepharose | 4.4 | 25.2 | 5.7 | 72.6 | 14-fach |
| UNO Q12 | 2.0 | 16.8 | 8.5 | 48.3 | 21-fach |
| Superdex 200 | 0.46 | 9.1 | 19.5 | 26.2 | 48-fach |

| | | | | | |
|---|---|---|---|---|---|
| ^{a)} Ein U der Amidase katalysiert die Bildung von 1 µmol Benzoesäure pro Minute und pro mg Protein unter Standardbedingungen. ^{b)} Nach Kultivierung bei 50°C, Zentrifugation der 1-Liter-Kultur (7g Zellmaterial (Nassgewicht)), zweimaliges Waschen mit Standardpuffer (pH 7,2) und Ultraschallaufschluss. | | | | | |

Mit dem beschriebenen Reinigungsverfahren wurde die enzymatisch aktive Amidase mit einer Ausbeute von 26,2 % erhalten. Die Spezifische Aktivität betrug 19,5 U/mg Protein bezogen auf Benzamid als Substrat. Die Amidaseaktivität wurde durch eine HPLC-Analyse der Produkte des enzymatisch umgesetzten Benzamids bestätigt.

### Beispiel 3: Bestimmung des Molekulargewichts der Amidase

Das Molekulargewicht der Amidase wurde mittels Gelfiltration an einer Superdex 200 Säule (Amersham) unter Verwendung eines 60 mM K-Na-Phosphatpuffers (pH 7,2), enthaltend 150 mM NaCl, bestimmt. Die Säule wurde mit Blue Dextran (2000 kDa), Sweet Potato β-Amylase (200 kDa), Yeast Alcohol Dehydrogenase (150 kDa), Bovine Serum Albumin (66 kaDa), Carbonic Anhydrase (29 kDa) und Cytochrome c (12,4 kDa) (Sigma Aldrich, Deutschland) kalibriert.

Das Molekulargewicht des nativen Proteins wurde so auf ca. 110 kDa bestimmt (siehe dazu Fig. 2, Bahn 2). Zur Überprüfung der enzymatischen Aktivität des gereinigten Proteins wurde ein Zymogram (Bahn 3) erstellt. Bahn 1 enthält den Molekulargewichtsstandard.

Demgegenüber wurde das Molekulargewicht des Enzyms mittels SDS-PAGE Elektrophorese auf 50 kDa bestimmt (siehe dazu Fig. 1). Die enzymatische Aktivität wurde anhand des Zymograms (Bahn 6, Rohextrakt und Bahn 7, gereinigtes Enzym) überprüft.

Aus den Ergebnissen kann geschlossen werden, dass die gereinigte Amidase bevorzugt als Dimer aus zwei identischen ca. 50 kDa großen Untereinheiten vorliegt.

Weiterhin wurde die gereinigte Amidase einer Endoproteinase Asp-N-Spaltung unterworfen. Im Anschluss kann aus dem ca. 110 kDa Protein ein Peptid mit der Masse 1482u isoliert werden, das mittels Edman-Abbau sequenziert wurde. Erhalten wurde die **SEQ ID No.2.**

### Beispiel 4: Bestimmung des isoelektrischen Punktes

Der isoelektrische Punkt wurde mittels isoelektrischer Fokussierung mit einem Novex Polyacrylamidgel (pH3-10) und unter Verwendung einer Novex Elektrophoresekammer anhand den Angaben des Herstellers (Novex, Invitrogen, Niederlande) bestimmt. Der isoelektrische Punkt der gereinigten Amidase wurde so mit pH 4,2 ermittelt.

### Beispiel 5: N-terminale Sequenzierung der gereinigten Amidase

Die gereinigte Amidase wurde vom SDS-PAGE Gel durch Elektroblotting auf eine PVDF blotting Membran überführt. Die Aminosäuresequenz des N-terminalen Endes des so erhaltenen Enzyms wurde mittels Edman-Abbau bestimmt. Erhalten wurde die **SEQ ID No. 1.**

Ein Sequenzvergleich mit anderen N-terminalen Enden bekannter Amidasen ergab keine signifikanten Homologien.

Weiterhin wurde die gereinigte trimere Amidase einer Endoproteinase Asp-N Spaltung unterworfen. Im Anschluss kann aus dem ca. 150 kDa Protein ein Peptid mit der Masse 1482u isoliert werden, dass mittels Edman-Abbau sequenziert wurde. Erhalten wurde die **SEQ ID No. 2.**

### Beispiel 6: Enzym-Assays

### Allgemeines: Assay zur Bestimmung der hydrolytischen Aktivität

Zur Durchführung des Hydrolyse-Assays wurde, sofern nicht anders erwähnt, eine 5 mM Benzamid-Lösung in 500 µl eines 60 mM K-Na-Phosphatpuffers, pH 7,2, verwendet. Die enzymatische Umsetzung erfolgte eine Stunde bei 70 °C unter Zugabe von 5 µg des Enzyms. Die Reaktion wurde durch Kühlung des Reaktionsansatzes auf Eis gestoppt. Die Konzentration der enzymatisch erhaltenen Produkte (Benzoesäure und Ammoniak) wurde mittels HPLC und spektrometrisch unter Verwendung des Ammoniak Kits Spectroquant 114752 (Merck, Deutschland) bestimmt.
Eine Unit (U) an Amidaseaktivität wird definiert als die Enzymmenge, die die Bildung von 1 µmol Benzoesäure pro Minute katalysiert.

### Assay zur Bestimmung der Acyltransferase Aktivität

Die Hydroxamsäure Bildung wird durch 10-minütige Inkubation der Amidase bei 70 °C in einer Mischung aus 20 mM K-Na-Phosphat Puffer, pH 7,2, 100 µl einer 50-100 mM Amid- und 0,7 M Hydroxylaminlösung (eingestellt auf pH 7,0 unter Zugabe von 10 M NaOH) bestimmt. Die Hydroxylaminlösung wurde dazu frisch präpariert. Nach 10-minütiger Inkubation bei 50 °C - 70 °C wurden die Reaktionsbehälter auf Eis gestellt und 1 ml einer sauren Eisenchloridlösung (0.1 M FeCl₃ in 50 ml einer 0.5 M HCl-Lösung) zugegeben. Die erhaltene Menge an Hydroxamat wurde spektrometrisch bei einer Wellenlänge von 500 nm bestimmt.

### Beispiel 6.1: Einfluss von Temperatur und pH-Wert

Die Temperaturabhängigkeit der Amidaseaktivität wurde unter Standardbedingungen bei pH 7.2 bestimmt. Dabei wurden die Reaktionstemperaturen zwischen 30 °C und 90 °C variiert.

Die Ergebnisse sind in Fig. 3 graphisch dargestellt, es wurde ein Temperaturoptimum bei ca. 70 °C ermittelt.

Weiterhin wurde die Temperaturstabilität der Enzyme zwischen 50 °C und 80 °C über einen längeren Zeitraum überprüft. Die isolierte Amidase zeigt eine ausgezeichnete thermische Stabilität bei 60 °C (obere Kurve, Fig. 5) und immer noch eine gute Stabilität bei 70 °C (mittlere Kurve, Fig. 5). Bei einer Reaktionstemperatur von 80 °C lässt die enzymatische Aktivität bei pH 7,2 allerdings schnell nach (untere Kurve, Fig. 5).

Das pH-Wert Optimum wurde anhand der Hydrolyse von Benzamid (5 mM) bei 70 °C in einem 50 mM Puffer aus Natriumacetat (für pH 2-5) oder Natriumphosphat (für pH 5-13), wobei der pH-Wert unter Verwendung von 10 M NaOH sukzessive bis auf pH 13 eingestellt wurde, durchgeführt.

Die Ergebnisse sind in Fig. 4 graphisch dargestellt, es wurde ein pH-Wert Optimum bei ca. 7 ermittelt.

### Beispiel 6.2: Einfluss von Metallen und Inhibitoren

Zur Untersuchung des Einflusses bestimmter Reagenzien und Ionen auf die Amidaseaktivität wurde eine Lösung enthaltend 5 µg der Amidase und das Reagenz oder Ion 1 h bei 22 °C in 50 mM Phosphat-Puffer vorinkubiert. Anschließend wurde eine 5 mM Lösung Benzamid in 50 mM Phosphat-Puffer zugegeben bis die Endkonzentration des Reagenz im Reaktionsansatz 1 mM betrug. Die Reaktion erfolgte 1 h bei 70 °C. Die katalytische Aktivität wurde wie oben beschrieben bestimmt.

Die Ergebnisse sind in Tabelle 2 zusammengefasst:

**Tab. 2:**

| **Reagenz, 1mM** | **Amidaseaktivität in %** |
|---|---|
| Cu²⁺ | 26 |
| Ni²⁺ | 42 |
| Zn²⁺ | 93 |
| Mg²⁺ | 90 |
| Ca²⁺ | 85 |
| Mn²⁺ | 98 |
| Fe²⁺ | 80 |
| Fe³⁺ | 96 |
| Ba²⁺ | 87 |
| Co²⁺ | 0 |
| EDTA | 100 |
| lodacetamid | 90 |
| Iodacetat | 22 |
| SDS | 100 |
| Triton X-100 | 100 |
| DTT | 120 |

### Beispiel 6.3: Untersuchung der Substratspezifität der isolierten Amidase

Die Substratspezifität der Amidase wurde bei drei unterschiedlichen Temperaturen unter Berücksichtigung des jeweils verwendeten Substrates getestet. Zur Hydrolyse von Aminosäureamiden wurde die Reaktionsmischung 3 h bei 30 °C umgesetzt, im Falle der aliphatischen und cyclischen Amide eine 1 h bei 50 °C und bei aromatischen Amidsubstraten 1 h bei 70 °C. Die enzymatische Aktivität wurde dann, wie oben beschrieben, bestimmt. Als Kontrolle wurde jeder Ansatz ohne Enzym verwendet.

Die getesteten Substrate und die dazugehörige Amidaseaktivität sind in Tabelle 3 wiedergegeben:

**Tab. 3**

| **Substrat** | **Spezifische Aktivität (µmol min⁻¹ mg⁻¹)** |
|---|---|
| **Aliphatische Amide** | |
| Formamid | 9.2 |
| Harnstoff | 17.5 |
| Acetamid | 15.7 |
| N-methylharnstoff | -- |
| Acrylamid | 26.8 |
| DL-Lactamid | 18.9 |
| Malonamid | 7.4 |
| Propionamid | 25.4 |
| Fumaranamid | -- |
| Isobutyramid | 13.5 |
| Methacrylamid | 23.5 |
| Succinamid | 4.0 |
| Pivalinamid | 14.9 |
| Adipamid | 12.0 |
| Hexanamid | 9.3 |
| Cyclohexanamid | 10.1 |

| **Aromatische Amide** | |
|---|---|
| Sulfanylamid | -- |
| 2-Aminobenzamid | 3.4 |
| 4-Aminobenzamid | 6.5 |
| Benzamid | 19.4 |
| o-Hydroxybenzamid | 7.4 |
| p-Hydroxybenzamid | 12.1 |
| N-Phenylharnstoff | -- |
| Acetanilid | -- |
| Benzylcarbamat | -- |
| o-To4ylamid | -- |
| m-Tolylamid | 12.7 |
| p-Tolylamid | 13.1 |
| 2-Phenylpropionamid | 15.8 |
| 3-Indolylacetamid | 7.56 |

| **Heterocyclen** | |
|---|---|
| Pyrazinamid | 11.2 |
| Nicotinamid | 11.6 |
| Isonicotinamid | 7.1 |

| **Aminosäureamide** | |
|---|---|
| L-Alaninamid | 10.5 |
| L-Methioninamid | 14.4 |
| L-Prolinamid | 13.0 |
| L-Valinamid | 13.0 |
| L-Leucinamid | 10.8 |
| L-Tryptophanamid | 10.3 |
| 2-Hydroxy-4-(Methylthio)-buttersäureamid | 5.7 |
| L-tert.-Leucinamid | 0.08 |

### Beispiel 6.4: Untersuchung der Enantioselektivität der isolierten Amidase

Dazu wurden 10 µg der gereinigten Amidase mit einer 5 mM racemischen 2-Phenylpropionamid Lösung 3 h bei 70 °C inkubiert, wobei das Endvolumen des Reaktionsansatzes 500 µl betrug. In 30-minütigen Abständen wurden dem Reaktionsansatz Proben entnommen und durch eine HPLC-Messung analysiert. Die Produktbestimmung und die Quantifizierung der R- und S- Stereoisomere des 2-phenylpropionamids wurden durch Injektion definierter Mengen der entsprechenden Reinsubstanzen als Standard ermittelt. Der Enantiomerenüberschuss (ee) wurde anhand des Peakflächenverhältnisses erhalten durch die chirale HPLC-Messung errechnet (ee^{p} = (S-R)/S+R); ee^{p}(%)= (S-R)/S+R) x 100; mit p = Produkt).

Für die HPLC Messungen wurde eine chirale Chirobiotic T Säule (Astec, Whippany, USA) benutzt. Als Solvent wurde Ethanol und 20 mM Kaliumphosphat-Puffer, pH 7,2 mit einem Volumenverhältnis von 20:80 eingesetzt. Die Eluation erfolgte bei Raumtemperatur mit einer Flussgeschwindigkeit von 0,8 ml min⁻¹. Die Detektion erfolgte bei einer Wellenlänge von 210 nm. Die Ammoniak-Konzentration wurde mit der Phenol-hypochlorid Methode unter Verwendung des Ammoniak Kits Spectroquant 114752 (Merck, Deutschland) untersucht, wobei die freigesetzte Ammoniakmenge spektrometrisch unter Verwendung von Ammoniumchlorid als Standard bestimmt wurde.

Die Ergebnisse der Messungen sind in Fig. 7 graphisch dargestellt (S -Enantiomer obere Kurve; R -Enantiomer untere Kurve). Die gereinigte Amidase ist im Hinblick auf 2-Phenylpropionamid als Substrat hoch S-selektiv. Nach 60 Minuten war der Wert für den Enantiomerenüberschuss ee^{p} größer 95, bei einem Umsatz von 50%.

## Patentansprüche

1. Mono- oder multimere Amidasen **dadurch gekennzeichnet, dass** die Amidase eine Aminosäuresequenz gemäß **SEQ ID No. 3** besitzt oder dass die Amidase eine Aminosäuresequenz mit einer Homologie von mindestens 80 % zu SEQ ID No. 3 besitzt und eine N-terminale Sequenz **SEQ ID No. 1** und/oder eine Sequenz **SEQ ID No. 2** enthält und die homologe Amidase in einem Temperaturbereich zwischen 30 und 85°C und einem pH-Bereich zwischen 4,5 und 10,0 enzymatisch aktiv ist.

2. Mono- oder multimere Amidasen gemäß Anspruch 1 **dadurch gekennzeichnet, dass** die Amidase eine N-terminale Sequenz **SEQ ID No. 1** und die **SEQ ID No. 2** enthält.

3. Amidasen gemäß einem der Ansprüche 1 bis 2 mit einem Molekulargewicht des nativen monomeren Enzyms zwischen 47 und 53 kDa.

4. Amidasen gemäß einem der Ansprüche 1 bis 3, **dadurch gekennzeichnet, dass** das Enzym aus thermophilen Bakterien erhältlich ist.

5. Amidasen gemäß einem der Ansprüche 1 bis 3, **dadurch gekennzeichnet, dass** das Enzym aus Actinomyceten erhältlich ist.

6. Amidasen gemäß einem der Ansprüche 1 bis 3, **dadurch gekennzeichnet, dass** das Enzym aus *Pseudonocardia thermophila* erhältlich ist.

7. Amidasen nach einem der Ansprüche 1 bis 6, **dadurch gekennzeichnet, dass** das Enzym als Monomer oder Dimer, bestehend aus zwei monomeren Amidaseeinheiten gemäß einem der Ansprüche 1 bis 6, vorliegt.

8. Nukleinsäuren codierend eine erfindungsgemäße Amidase gemäß einem der Ansprüche 1 bis 7, **dadurch gekennzeichent, dass** die Nukleinsäure eine Sequenz gemäß **SEQ ID No. 4** oder eine Nukleotidsequenz mit einer Homologie von über 90 % dazu besitzt.

9. Verwendung von Amidasen gemäß einem der vorherigen Ansprüche zur Hydrolyse von Amiden oder zur Acylierung.

10. Verwendung von Amidasen gemäß Anspruch 9 zur Hydrolyse von aliphatischen Amiden, aromatischen Amiden, cyclischen Amiden, heterocyclischen Amiden oder Aminosäureamiden.

11. Verwendung von Amidasen gemäß Anspruch 10 zur enantioselektiven Hydrolyse von Amiden.

12. Verwendung von Amidasen gemäß Anspruch 11 zur Herstellung von S-stereoisomeren Säuren.

13. Verfahren zur enzymatisch-katalytischen Hydrolyse von Amiden **dadurch gekennzeichnet, dass** die Reaktion durch eine Amidase gemäß den Ansprüchen 1 bis 7 katalysiert wird.

14. Verfahren gemäß Anspruch 13, **dadurch gekennzeichnet, dass** die Umsetzung bei einer Temperatur zwischen 30 °C und 85 °C durchgeführt wird.

15. Verfahren gemäß Anspruch 14, **dadurch gekennzeichnet, dass** die Umsetzung bei einer Temperatur zwischen 50 °C und 75 °C durchgeführt wird.

16. Verfahren gemäß einem der Ansprüche 13 bis 15, **dadurch gekennzeichnet, dass** die Umsetzung bei einem pH-Wert zwischen 3,5 und 11,5 erfolgt.

## Claims

1. Monomeric or multimeric amidases **characterized in that** the amidase possesses an amino acid sequence in accordance with **SEQ ID No. 3** or **in that** the amidase possesses an amino acid sequence having a homology of at least 80% with SEQ ID No. 3 and comprises an N-terminal sequence **SEQ ID No. 1** and/or a sequence **SEQ ID No. 2**, and the homologous amidase is enzymatically active in a temperature range between 30 and 85°C and in a pH range between 4.5 and 10.0.

2. Monomeric or multimeric amidases **characterized in that** the amidase contains an N-terminal sequence **SEQ ID No. 1** and **SEQ ID No. 2**.

3. Amidases according to one of Claims 1 to 2 having a molecular weight of the native monomeric enzyme between 47 and 53 kDa.

4. Amidases according to one of Claims 1 to 3, **characterized in that** the enzyme is obtainable from thermophilic bacteria.

5. Amidases according to one of Claims 1 to 3, **characterized in that** the enzyme is obtainable from Actinomycetes.

6. Amidases according to one of Claims 1 to 3, **characterized in that** the enzyme is obtainable from *Pseudonocardia thermophila.*

7. Amidases according to one of Claims 1 to 6, **characterized in that** the enzyme is present as monomer or dimer, consisting of two monomeric amidase units according to one of Claims 1 to 6.

8. Nucleic acids coding for an inventive amidase according to one of Claims 1 to 7, **characterized in that** the nucleic acid has a sequence according to **SEQ ID No. 4** or a nucleotide sequence having a homology of greater than 90% therewith.

9. Use of amidases according to one of the preceding claims for the hydrolysis of amides or for acylation.

10. Use of amidases according to Claim 9 for the hydrolysis of aliphatic amides, aromatic amides, Cyclic amides, heterocyclic amides or amino acid amides.

11. Use of amidases according to Claim 10 for the enantioselective hydrolysis of amides.

12. Use of amidases according to Claim 11 for preparing *S*-stereoisomeric acids.

13. Method for the enzymatically catalyzed hydrolysis of amides, **characterized in that** the reaction is catalyzed by an amidase according to Claims 1 to 7.

14. Method according to Claim 13, **characterized in that** the reaction is carried out at a temperature between 30°C and 85°C.

15. Method according to Claim 14, **characterized in that** the reaction is carried out at a temperature between 50°C and 75°C.

16. Method according to one of Claims 13 to 15, **characterized in that** the reaction proceeds at a pH between 3.5 and 11.5.

## Revendications

1. Amidases mono- ou multimères, **caractérisées en ce que** l'amidase a une séquence d'acides aminés selon SEQ ID N°3, ou que l'amidase a une séquence d'acides aminés présentant avec SEQ ID N°3 une homologie d'au moins 80 %, et contient une séquence N-terminale SEQ ID N°1 et/ou une séquence SEQ ID N°2, et que l'amidase homologue est enzymatiquement active sur une plage de températures de 30 à 85°C et sur une plage de pH de 4,5 à 10,0.

2. Amidases mono- ou multimères selon la revendication 1, **caractérisées en ce que** l'amidase contient une séquence N-terminale SEQ ID N°1 et SEQ ID N°2.

3. Amidases selon l'une des revendications 1 à 2, ayant une masse moléculaire de l'enzyme monomère native comprise entre 47 et 53 kDa.

4. Amidases selon l'une des revendications 1 à 3, **caractérisées en ce que** l'enzyme peut être obtenue à partir de bactéries thermophiles.

5. Amidases selon l'une des revendications 1 à 3, **caractérisées en ce que** l'enzyme peut être obtenue à partir d'Actinomycètes.

6. Amidases selon l'une des revendications 1 à 3, **caractérisées en ce que** l'enzyme peut être obtenue à partir de *Pseudonocardia thermophila.*

7. Amidases selon l'une des revendications 1 à 6, **caractérisées en ce que** l'enzyme se présente sous forme monomère ou dimère, constituée de deux motifs amidases monomères selon l'une des revendications 1 à 6.

8. Acides nucléiques codant pour une amidase selon l'invention selon l'une des revendications 1 à 7, **caractérisés en ce que** l'acide nucléique possède une séquence selon SEQ ID N°4 ou une séquence nucléotidique ayant avec cette dernière une homologie supérieure à 90 %.

9. Utilisation d'amidases selon l'une des revendications précédentes pour l'hydrolyse d'amides ou pour l'acylation.

10. Utilisation d'amidases selon la revendication 9 pour l'hydrolyse d'amides aliphatiques, d'amides aromatiques, d'amides cycliques, d'amides hétérocycliques et d'amides d'acides aminés.

11. Utilisation d'amidases selon la revendication 10 pour l'hydrolyse énantio-sélective d'amides.

12. Utilisation d'amidases selon la revendication 11 pour préparer des acides S-stéréoisomères.

13. Procédé d'hydrolyse d'amides catalysées par une enzyme, **caractérisé en ce que** la réaction est catalysée par une amidase selon les revendications 1 à 7.

14. Procédé selon la revendication 13, **caractérisé en ce que** la réaction est mise en oeuvre à une température comprise entre 30 et 85°C.

15. Procédé selon la revendication 14, **caractérisé en ce que** la réaction est mise en oeuvre à une température comprise entre 50 et 75°C.

16. Procédé selon l'une des revendications 13 à 15, **caractérisé en ce que** la réaction a lieu à un pH compris entre 3,5 et 11,5.
